# EUROPEAN PATENT APPLICATION

(11) **EP 3 979 252 A1**
(43) Date of publication of application: **06.04.2022**
(21) Application number: 20813247.2
(22) Date of filing: 29.05.2020
(51) Int. Cl.: G16B 30/00

(54) **INFORMATION PROCESSING DEVICE, INFORMATION PROCESSING METHOD, AND INFORMATION PROCESSING PROGRAM**

(30) Priority: 31.05.2019 JP 2019102716
(71) Applicant: NEC Corporation, 108-8001 Tokyo (JP)
(72) Inventor: ASOGAWA, Minoru, Tokyo 108-8001 (JP)
(74) Representative: Betten & Resch
(86) International application number: PCT/JP2020/021351
(87) International publication number: WO 2020/241829

(57) **Abstract**

To increase reliability in DNA profiling. An error sequence generated by artifact upon PCR and a generation probability thereof are obtained by a preliminary experiment and stored in a storage part. A sequence analysis result in a DNA profiling is obtained. The storage part is referred while regarding the read sequences as the true sequence for each of read sequences listed in the analysis result so as to acquire an associated error sequence as a prospected error sequence and obtain a value as a prospected read number by multiplying the generation probability of the associated error sequence with the read number of each of the read sequences. In addition, a read sequence identical with the prospected error sequence among the read sequences listed in the analysis result is retrieved. It is determined that a retrieved read sequence is an error sequence in a case where the read number of the retrieved read sequence matches with the prospected read number.

## Description

### FIELD

### (REFERENCE TO RELATED APPLICATION)

The disclosure is based on the priority of Japanese patent application No. 2019-102716 (filed on May 31, 2019), and the entire contents of the same application are incorporated by reference into the application. The disclosure relates to an information processing apparatus, an information processing method and an information processing program. Particularly, the disclosure relates to an information processing apparatus, an information processing method and an information processing program for DNA profiling.

### BACKGROUND

DNA profiling using microsatellites has been performed. The microsatellites include repeat sequences, thus a phenomenon occurs upon PCR amplification, in which the number of repeats is increased or reduced when compared with an original sequence. Such phenomenon is referred to as "stutter", and provides a negative influence on reliability in the DNA profiling. Therefore, various technologies have been developed in order to eliminate the influence by the stutter. For example, Patent Literature 1 (PTL 1) discloses a technology in which the height of a stutter peak is estimated.

In addition, in a recent DNA profiling, isoalleles which have the same sequence length, but have different nucleotide sequences are identified using a technology referred to as "NGS (next generation sequencing)". The DNA profiling using NGS reads not only true sequences which have been correctly amplified, but also a stutter sequence generated by stutter. However, the isoalleles are determined by disregarding the stutter sequence in a manner referred to as "stutter filter". That is, the stutter filter is a filter by which sequences having a read number of a ratio less than a threshold are uniformly disregarded. The read number of the stutter sequence would be significantly smaller than the read number of the true sequences, resulting in disregarding of the stutter sequence.

### CITATION LIST

### PATENT LITERATURE

PTL 1: Tokkai JP 2006-163720A

### SUMMARY

### TECHNICAL PROBLEM

The following analysis is provided from an aspect of the disclosure. Herein, the disclosure of the PTL is incorporated by reference.

A sample subjected to DNA profiling sometime includes DNAs of multiple persons at different ratios. For example, a sample obtained from a crime scene includes a lot of DNA from a victim and a little of DNA from a criminal offender (hereinafter, referred to as "criminal"). In a case where such sample is analyzed by NGS, the read number of the true sequence from the criminal would be small. If the above described stutter filter is applied thereto, the true sequence from the criminal would be disregarded.

Herein, the technology disclosed in PTL 1 is useful for setting a threshold for the stutter filter, but does not provide any solutions to the above problem.

Accordingly, it is a purpose of the disclosure to provide an information processing apparatus, an information processing method and an information processing program which may contribute to improve the reliability in DNA profiling.

### SOLUTION TO PROBLEM

According to a first aspect, there is provided an information processing apparatus, comprising:
a storage part that stores, for each of isoalleles of a microsatellite which are identified in DNA profiling, a true sequence correctly amplified by PCR, an error sequence incorrectly amplified upon PCR, and a generation probability of the error sequence in association with each other;
an analysis result acquiring part that acquires an analysis result in which read sequences which are read by subjecting a sample to PCR and sequence analysis and read numbers of the read sequences are listed in association with each other;
a prospect part that refers to the storage part while regarding the read sequences as a true sequence for each of the read sequences listed in the analysis result so as to acquire an associated error sequence as a prospected error sequence, and obtains a value as a prospected read number by multiplying the generation probability of the associated error sequence with the read number of each of the read sequences;
a determination part that retrieves a read sequence identical with the prospected error sequence among the read sequences listed in the analysis result, and determines that a retrieved read sequence as an error sequence in a case where the read number of the retrieved read sequence matches with the prospected read number.

According to a second aspect, there is provided an information processing method, including:
an analysis result acquiring step of acquiring an analysis result in which read sequences which are read by subjecting a sample to PCR and sequence analysis and read numbers of the read sequences are listed in association with each other;
a prospect step of referring to a storage part that stores, for each of isoalleles of a microsatellite which are identified in DNA profiling, a true sequence correctly amplified by PCR, an error sequence incorrectly amplified upon PCR, and a generation probability of the error sequence in association with each other , while regarding the read sequences as a
true sequence for each of the read sequences listed in the analysis result so as to acquire an associated error sequence as a prospected error sequence, and obtaining a value as a prospected read number by multiplying the generation probability of the associated error sequence with the read number of each of the read sequences;
a determination step of retrieving a read sequence identical with the prospected error sequence among the read sequences listed in the analysis result, and determining that a retrived read sequence as an error sequence in a case where the read number of the retrived read sequence matches with the prospected read number.

According to a third aspect, there is provided
an information processing program causing a computer to execute: an analysis result acquiring process of acquiring an analysis result in which read sequences which are read by subjecting a sample to PCR and sequence analysis and read numbers of the read sequences are listed in association with each other ;
a prospect process of referring to a storage part that stores, for each of isoalleles of a microsatellite which are identified in DNA profiling, a true sequence correctly amplified by PCR, an error sequence incorrectly amplified upon PCR, and a generation probability of the error sequence in association with each other, while regarding the read sequences as a true sequence for each of the read sequences listed in the analysis result so as to acquire an associated error sequence as a prospected error sequence, and obtaining a value as a prospected read number by multiplying the generation probability of the associated error sequence with the read number of each of the read sequences;
a determination process of retrieving a read sequence identical with the prospected error sequence among the read sequences listed in the analysis result, and determining that a retrived read sequence as an error sequence in a case where the read number of the retrived read sequence matches with the prospected read number.

### ADVANTAGEOUS EFFECTS OF INVENTION

According to each aspect of the disclosure, there are provided an information processing apparatus, an information processing method and an information processing program that contribute to improve the reliability in DNA profiling.

### BRIEF DESCRIPTION OF THE DRAWINGS

Figure 1 is an explanatory view of one outline of the disclosure.
Figure 2 is an explanatory view of one outline of the disclosure.
Figure 3 is an explanatory view of one outline of the disclosure.
Figure 4 is an explanatory view of one outline of the disclosure.
Figure 5 is an explanatory view of one outline of the disclosure.
Figure 6 is a block diagram showing a configuration of a computer as an information processing apparatus 100 of Example embodiment 1.
Figure 7 is a diagram showing one example information stored in a storage part 110.
Figure 8 is a sequence diagram showing a flow of processes by the information processing apparatus 100 of Example embodiment 1.
Figure 9 is a block diagram showing a configuration of a computer as an information processing apparatus 100 of Example embodiment 2.
Figure 10 is an explanatory view of an effect by the information processing apparatus 100 of Example embodiment 2.
Figure 11 is an explanatory view of an effect by the information processing apparatus 100 of Example embodiment 2.

### MODES

A preferable example embodiment of the disclosure is explained in detail while referring to drawings. Herein, reference signs appended to the following disclosure expediently appended to each element as one example for an aid for understanding, it is not intended to limit the disclosure to the configuration illustrated in the drawings. In addition, a connection line between blocks in drawings includes both of bidirectional and monodirectional connections. Further, although omitted in block diagrams and the like disclosed in the application, an input port and an output port are provided on an input end and an output end of each connection line, respectively. The same is applied to an input/output interface.

### [Terms]

First, terms used in the disclosure are explained. Herein, for example, STRBase (Short Tandem Repeat DNA Internet DataBase, https://strbase.nist.gov/index.htm) should be also referenced for explanation of each term.

"DNA (deoxyribonucleic acid)" refers to a chemical compound comprising adenine (A), guanine (G), cytosine (C) and thymine (T), but also refers to "genetic information" of individual persons in the application. For example, "DNA profiling" may be interchanged by personal profiling based on genetic information, and "DNA of victim" may be interchanged by genetic information of the victim.

"Microsatellite" refers to a repeat sequence itself and a region, a tract, a site, a position which comprise the repeat sequence, but also refers to a comprehensive name of loci in the application.

"Locus (loci)" refers to a position on a chromosome. The locus may be referred to as a marker name, such as CSF1PO, D1S1656 and the like.

"Isoalleles" refers to a type of variants provided on each locus. On the STRBase, it is referred to as Allele (Repeat #): 11', and the like.

"Sequence" refers to a sequence of nucleotide bases. In addition, "repeat sequence (repetitive sequence)" is also called as STR (short tandem repeat). In a case where a sequence of 2 or more nucleotide bases is regarded as one unit, the "repeat sequence" comprises plural times of repeats of the unit(s) (single or multiple). On the STRBase, it is also referred to as "Repeat Structure". For example, a repeat sequence indicated by "[CCTA]1[TCTA]10" refers to a sequence in which a unit [TCTA] tandemly repeats 10 times subsequent to a unit [CCTA]. Herein, "[CCTA]1[TCTA]10" may be also indicated as "[TAGA]10[TAGG]1" (i.e., antiparallel (complementary) sequence), and they are regarded as identical in STR analysis. Herein, there is also a case where 3 to 5 nucleotides are regarded as one repeat unit.

"True sequence" refers to a sequence of a case where a repeat sequence is correctly amplified by PCR (Polymerase Chain Reaction), and "error sequence" refers to a sequence of an incorrectly amplified repeat sequence upon PCR. Herein, "error" includes stutter, indel, nucleotide substitution. That is, the "true sequence" refers to a sequence of which a sequence included in a sample is amplified without any artifacts, such as stutter, etc. Herein, a sequence included in the sample itself may be referred to as both of the "true sequence" and an "original sequence", but has the same sequence as itself.

"Stutter" refers to a phenomenon that the repeat number is increased or reduced compared with an original sequence upon PCR amplification. Herein, a sequence in which the stutter occurs is referred to as "stutter sequence".

"Indel (insertion/deletion)" refers to a phenomenon that one or more nucleotide base is inserted into/deleted from an original sequence, and includes indel occurring upon PCR amplification and indel due to artifact upon sequence analysis. Herein, "indel" in the application is used in a different meaning from gene polymorphism within an original sequence (so called insertion/deletion polymorphism). Herein, a sequence in which the indel occurs is referred to as "indel sequence".

"Nucleotide substitution" refers to a phenomenon that one or more nucleotide base in an original sequence is substituted with another nucleotide base, and includes nucleotide substitution occurring upon PCR amplification and nucleotide substitution due to artifact upon sequence analysis. Herein, "nucleotide substitution" in the application is used as a different meaning from so-called point mutation. Herein, a sequence in which the nucleotide substitution occurs is referred to as "nucleotide substitution sequence".

"Generation probability of the error sequence" has a similar meaning as those of generation frequency of error, a relative amount of a fragment which is incorrectly amplified upon PCR, and generation frequency of artifact upon sequence analysis.

"Sequential analysis" refers to an analysis for determining a nucleotide sequence, and also refers to as "DNA sequencing". In addition, "sequential analysis" is also expressed in a context of "reading" a sequence. Herein, the above terms "true sequence", "error sequence" are also sequences that are determined by the sequential analysis. However, in the application, these sequences have been previously determined by experiments. On the other hand, the term "read sequence" refers to a sequence to be actually read upon DNA profiling, (i.e., raw data).

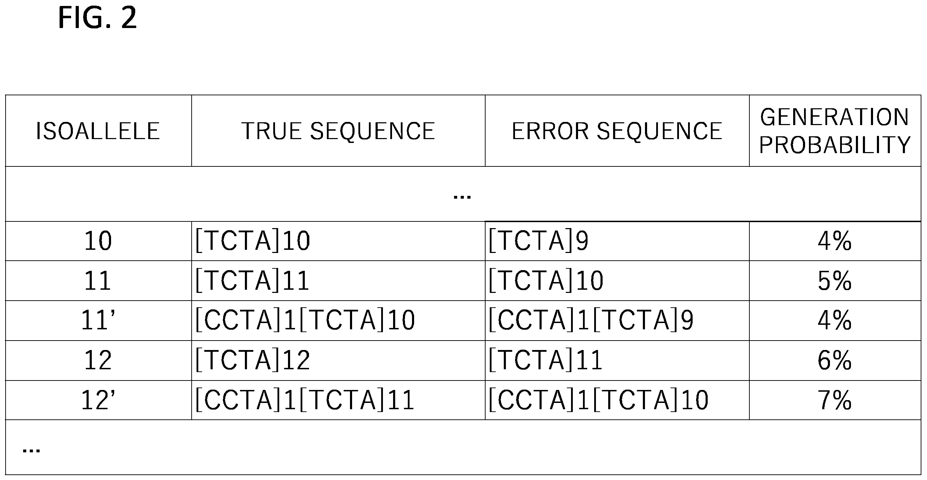

Herein, in the application, it is preferable that a technology referred to as NGS (next generation sequencing) is applied to the sequential analysis. NGS includes a nanopore sequencing (for example, see WO2016/075204), a cluster generation sequencing (for example, see WO2014/108810), etc. Any types of sequential analysis may be applied to the application, in which DNA fragments are amplified by PCR, sequences of the amplified DNA fragments are read respectively, and then the number of reading of the same sequence (i.e., "read number") is obtained. In other words, the sequential analysis of the application may be applied if it is possible to finally obtain an analysis result, for example, as shown in Figure 2. Herein, the "read number" corresponds to a meaning of "depth of coverage" in a field of NGS, and the like.

### [One outline of the disclosure]

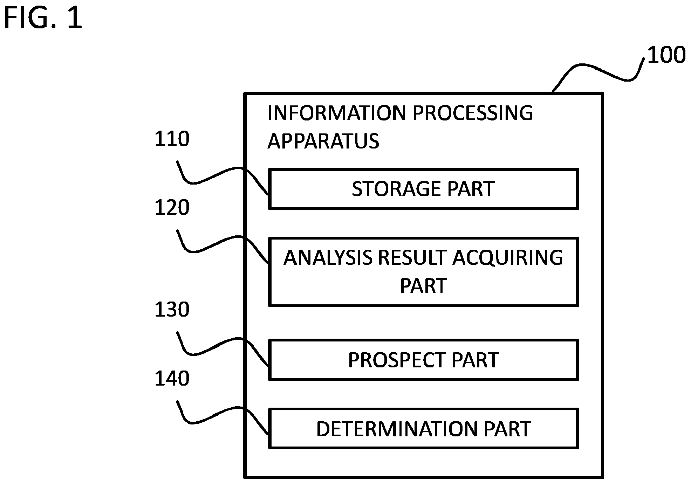

Next, one outline of the disclosure is explained while referring to Figures 1 to 5. Herein, in order to simplify the explanation, a part of information is simplified into a configuration different from actual information. As illustrated in Figure 1, an information processing apparatus 100 comprises a storage part 110, an analysis result acquiring part 120, a prospect part 130 and a determination part 140.

The storage part 110 stores, for each of isoalleles of a microsatellite which are identified in DNA profiling, a true sequence correctly amplified by PCR, an error sequence incorrectly amplified upon PCR, and a generation probability of the error sequence in association with each other. For example, as illustrated in Figure 2, the storage part 110 stores, for ISOALLELE: 10, TRUE SEQUENCE: TCTA 10, ERROR SEQUENCE: TCTA 9, and GENERATION PROBABILITY: 4%. Herein, Figure 2 indicates information of LOCUS: D1S1656. The true sequence of each isoallele may be obtained by referring to STRBase, etc. In addition, the error sequence indicated in Figure 2 is a sequence that one unit [TCTA] is reduced (deleted) from the true sequence due to stutter. The error sequence and the generation probability may be obtained from a preliminary experiment and previously stored in the storage part 110.

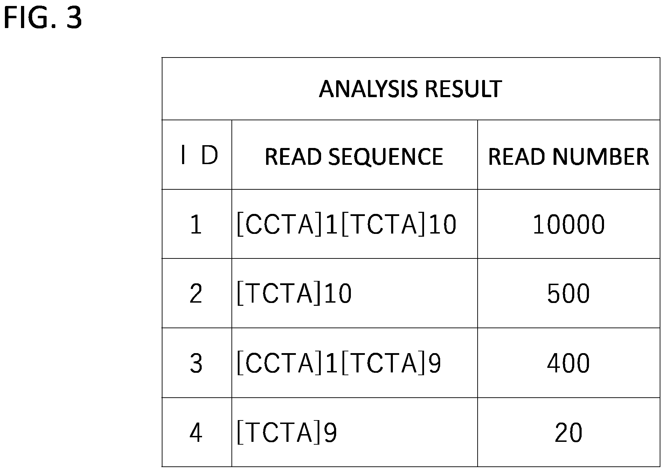

The analysis result acquiring part 120 acquires an analysis result in which read sequences which are read by subjecting a sample to PCR and sequence analysis and read number of each of the read sequences are listed in association with each other. For example, the analysis result acquiring part 120 acquires an analysis result illustrated in Figure 3. The analysis result is information acquired upon DNA profiling. For example, the analysis result acquiring part 120 acquires the analysis result from a sequence apparatus (not illustrated) connected in a communicable manner to the information processing apparatus 100.

The prospect part 130 refers to the storage part 110 while regarding the read sequences as the true sequence for each of the read sequences listed in the analysis result. Then the prospect part 130 acquires an associated error sequence as a prospected error sequence, and obtains a value as a prospected read number by multiplying the generation probability of the associated error sequence with the read number of each of the read sequences.

For example, the prospect part 130 searches the storage part 110, using READ SEQUENCE: [CCTA 1][TCTA 10] as a search key, for a true sequence identical with the read sequence. In the example illustrated in Figure 2, a true sequence of ISOALLELE: 11' is retrieved as an identical sequence. Herein, the prospect part 130 acquires ERROR SEQUENCE: [CCTA 1][TCTA 9] of ISOALLELE: 11' from the storage part 110. This ERROR SEQUENCE: [CCTA 1][TCTA 9] is a sequence prospected to be incorrectly amplified upon PCR amplification of the READ SEQUENCE: [CCTA 1][TCTA 10], thus the process by the prospect part 130 may be also referred to as a process of obtaining an error sequence from the storage part 110. In addition, the prospect part 130 acquires GENERATION PROBABILITY: 4% of ERROR SEQUENCE: [CCTA 1][TCTA 9] from the storage part 110. Then the prospect part 130 multiplies the obtained GENERATION PROBABILITY: 4% with the READ NUMBER "10000" of the READ SEQUENCE: [CCTA 1][TCTA 10] to calculate PROSPECTED READ NUMBER: 400. The prospected read number is a value prospected as the read number of [CCTA 1][TCTA 9] under a situation where the READ SEQUENCE: [CCTA 1][TCTA 10] is read 10000 times.

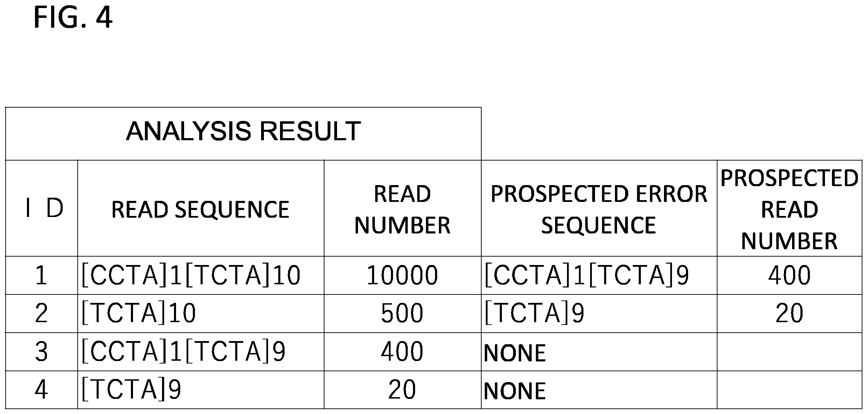

Furthermore, the prospect part 130 executes the same process for READ SEQUENCE: [TCTA 10], and obtains PROSPECTED ERROR SEQUENCE: [TCTA 9] and PROSPECTED READ NUMBER: 20. With respect to READ SEQUENCEs: [CCTA 1][TCTA 9] and [TCTA 9], there are no identical sequences in the true sequences in the storage part 110, thus the prospect part 130 determines PROSPECTED ERROR SEQUENCE: NONE and terminates its process. These processes by the prospect part 130 is conceptionally illustrated in Figure 4.

The determination part 140 retrieves a read sequence identical with the prospected error sequence among the read sequences listed in the analysis result, and determines that the retrieved read sequence as the error sequence in a case where the read number of the retrieved read sequence matches with the prospected read number.

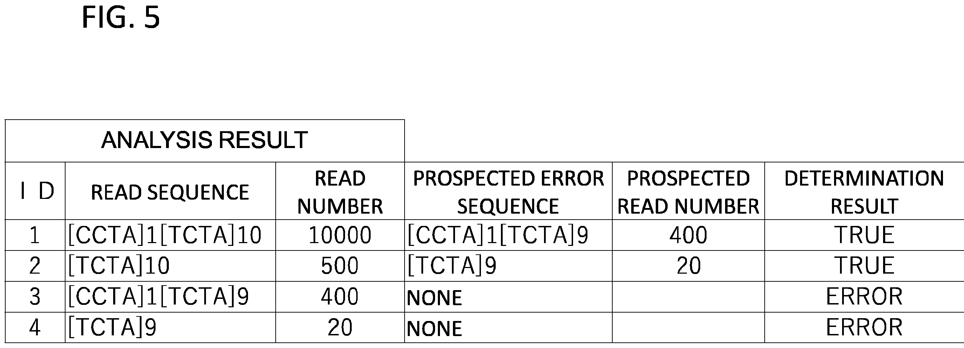

For example, in the example illustrated in Figure 4, using PROSPECTED ERROR SEQUENCE: [CCTA 1][TCTA 9] as a search key, the determination part 140 retrieves an identical READ SEQUENCE (ID: 3) among the read sequences listed in the analysis result. Herein, since the PROSPECTED READ NUMBER of PROSPECTED ERROR SEQUENCE: [CCTA 1][TCTA 9] is 400 and the READ NUMBER of ID: 3 is also 400, thus the determination part 140 determines that they match one another and determines that ID: 3 is an error sequence (ERROR). In addition, the determination part 140 similarly determines that ID: 4 is also an error sequence. Herein, with respect to IDs: 1, 2, they are not determined as the error sequence, thus the determination part 140 determines that they are true sequences (TRUE). These processes by the determination part 140 is conceptionally illustrated in Figure 5.

Herein, an effect exerted by the above information processing apparatus 100 is explained while comparing with a case of applying a stutter filter. For example, with respect to LOCUS: D1S1656, it is known that the stutter occurs at a probability of approximately 7%. The stutter filter is a filter for eliminating an effect by the stutter, thus a threshold exceeding 7% (for example 10%) is set as the stutter filter. In a case where 10% is set as the threshold for the stutter filter, in the analysis result of Figure 3,
read sequences having a read number of 1000 or less would be disregarded since the read number of ID: 1 which may be recognized as a true sequence is 10000. That is, in a case where the stutter filter is applied, ID: 2 would be also determined as an error sequence and disregarded. On the other hand, in the information processing apparatus 100 of the disclosure, ID: 2 is determined as a true sequence as indicated in Figure 5.

Such difference provides a significant effect in a case where a sample to be applied to DNA profiling includes DNAs of multiple persons at different rates. For example, a case is considered where a sample which had been obtained from a crime scene and supposed to include a little amount of DNA of a criminal was subjected to PCR and sequential analysis, and then the analysis result illustrated in Figure 3 has been obtained.

If the stutter filter is applied, IDs: 2 to 4 would be determined as the error sequence and disregarded as described above, and only ID: 1 would be determined as the true sequence. ID: 1 would be determined as being derived from a victim, and resulting in a determination that the sample would not include DNA of the criminal.

On the other hand, in the information processing apparatus 100 of the disclosure, ID: 2 is determined as the true sequence. Herein, the read number of ID: 2 is significantly less than the read number of ID: 1, thus it is determined that ID: 2 is derived from a person different from ID: 1. That is, according to the information processing apparatus 100 of the disclosure, ID: 2 is determined as being derived from a criminal.

As described above, according to the information processing apparatus 100 of the disclosure, reliability in DNA profiling may be improved.

### [Example embodiment 1]

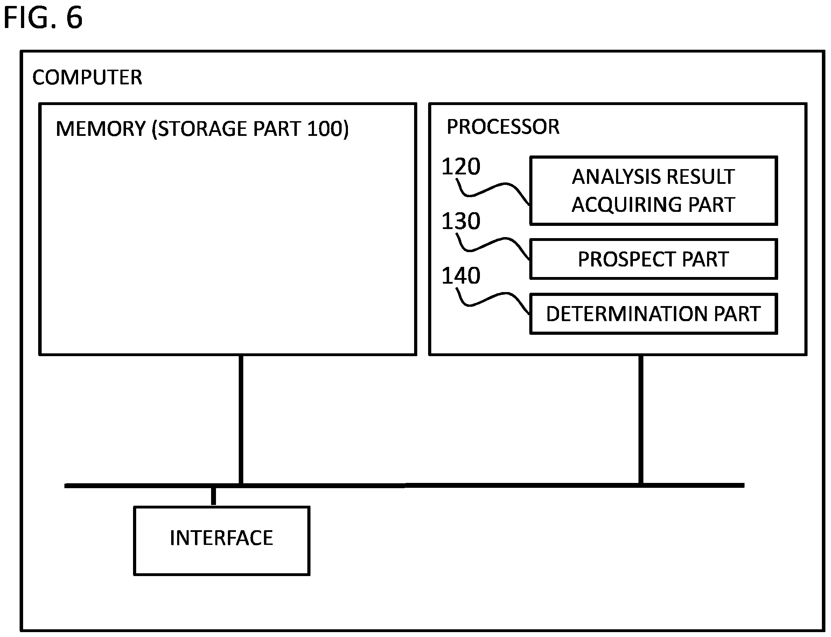

In the following description, the information processing apparatus 100 explained in the above one outline is explained more concretely. An information processing apparatus 100 of an example embodiment 1 is realized as a computer comprising a memory, a processor and an interface as illustrated in Figure 6. The memory is a ROM (read only memory), a RAM (random access memory), a cache memory, and the like, that stores a program, etc., for controlling processes by the entire information processing apparatus 100. In the first example embodiment, the memory also stores information like as the storage part 110, thus the memory is referred to as "storage part 110" hereinafter.

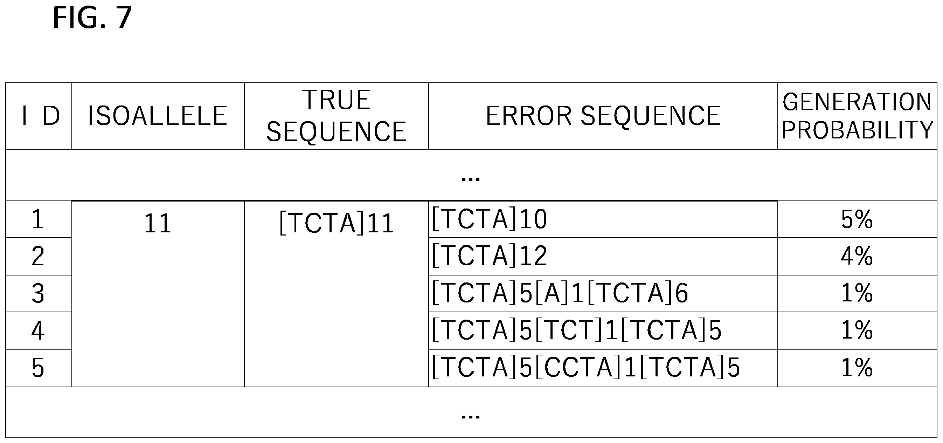

Information stored in the storage part 110 may include a plurality of error sequences for one isoallele as illustrated in, for example, Figure 7. In Figure 7, the error sequence of ID: 1 is a stutter sequence in which one unit: [TCTA] is deleted. The error sequence of ID: 2 is a stutter sequence in which one unit: [TCTA] is inserted. The error sequence of ID: 3 is an indel sequence in which one nucleotide base: A is inserted subsequent to 5 repeats of unit: [TCTA]. The error sequence of ID: 4 is an indel sequence in which a nucleotide base: A in 6th unit: [TCTA] is deleted. The error sequence of ID: 5 is a nucleotide substitution sequence in which an initial nucleotide base: T in 6th unit: [TCTA] is substituted by C. The error sequences and their generation probabilities are obtained by performing a preliminary experiment in which DNA fragment whose sequence has been determined is subjected to PCR amplification. These items of information are previously stored in the storage part 110 before actually carrying out DNA profiling. Herein, the generation probability would be changed due to PCR condition (type of polymerase, salt concentration, cycle number, and the like) sample condition (contamination and the like), and type of sequential analysis, thus it is preferable to precisely define these conditions. In addition, the storage part 110 stores not only information relating to LOCUS: D1S1656, but also information relating to the other locus (CSF1PO, D12S391, etc.). Herein, the information stored in the storage part 110 may be created by using machine learning technology, for example, as disclosed in JP patent No. 5299267 B.

The processor is configured to comprise CPU (Central Processing Unit) and a chip, and reads out programs from the storage part to realize processing modules required for the disclosure. The computer of the example embodiment 1 realizes the analysis result acquiring part 120, the prospect part 130 and the determination part 140 as the processing modules, which are explained in the above one outline. In the following description, points different from the above one outline are explained.

The analysis result acquiring part 120 acquires not only the analysis result relating to LOCUS: D1S1656 as illustrated in Figure 3, but also analysis results relating to the other loci (CSF1PO, D12S391, and the like) (not illustrated). Herein, such analysis results may include, for each true sequence, not only error sequences incorrectly amplified upon PCR, but also indel sequence(s) and nucleotide substitution sequence(s) due to artifact upon sequential analysis. Herein, it is prospected that the read number of the indel sequence and the nucleotide substitution sequence which are generated due to the artifact upon the sequential analysis, thus the analysis result acquiring part 120 may exclude read sequence(s) having a read number less than a predetermined threshold (for example, less than 10) from the analysis result.

The determination part 140 determines that a read sequence is an error sequence in a case where a read number of a read sequence identical with a prospected error sequence matches with a prospected read number. Herein, the term "match" includes not only a case where the read number of the read sequence is completely consistent with the prospected read number, but also a case where the read number of the read sequence is consistent with the prospected read number at a reasonable extent. For example, in a case where the read number of the read sequence is within ±50% of the prospected read number, the determination part 140 may determine that they match one another. In addition, in a case where the read number of the read sequence is less than the prospected read number, the determination part 140 determines that they match each other. Herein, a range and a threshold in a concept of "match" may be variously set based on, for example, a purpose of DNA profiling, such as paternity test, determination of a criminal, etc., and PCR condition, such as sample condition, PCR condition, etc.

Herein, the determination result provided by the determination part 140 is output and displayed on a display and the like via the interface.

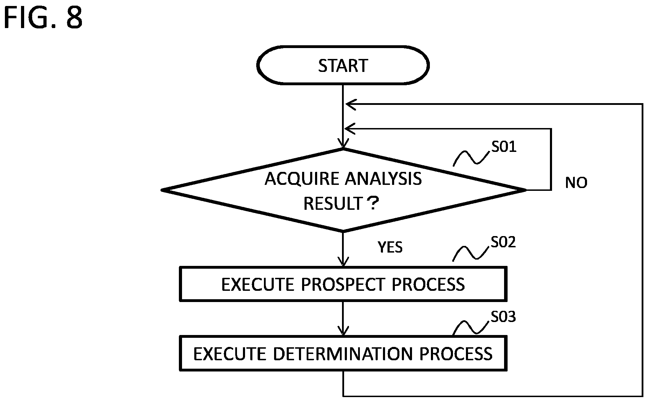

In the following description, a flow of a sequential process by the information processing apparatus 100 of the example embodiment 1 is explained. As illustrated in Figure 8, when the analysis result acquiring part 120 acquires an analysis result (step S01: YES), the prospect part 130 executes a prospect process of obtaining a prospected error sequence and a prospected read number (step S02). In addition, the determination part 140 executes a determination process of retrieving a read sequence identical with the prospected error sequence, and determining that the retrieved read sequence is the error sequence in a case where the read number of the retrieved read sequence matches with the prospected read number (step S03).

As described above, the information processing apparatus 100 of the example embodiment 1 may eliminate, from the DNA profiling, effects due to not only stutter sequence, but also indel sequence and nucleotide substitution sequence generated due to artifact upon PCR.

### [Example embodiment 2]

In an aspect of reliability in DNA profiling, peak height balance in the analysis result would be also regarded as important. An analysis result having imbalanced peak height would provide poor reliability in profiling of a person of heterozygous. Therefore, in the following description, an information processing apparatus 100 capable of overcoming a problem relating to imbalanced peak height is explained as an example embodiment 2. Herein, with respect to the peak height balance, see also for example, Kagaku to Seibutsu 55(8): 559-565 (2017), "Discrimination among Individuals with Analysis of DNA Profiles: Application of New Forensic Science Technologies Using Microbiota Profiling".

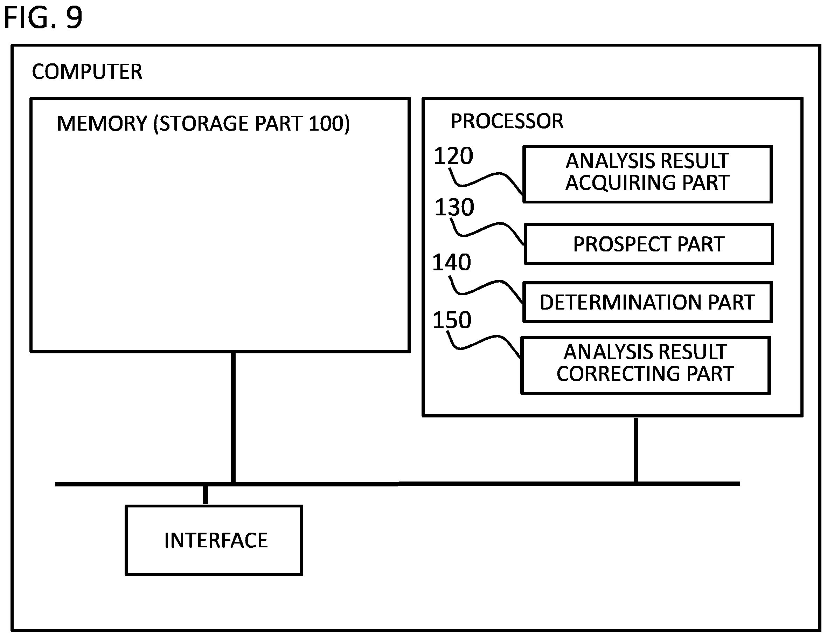

As illustrated in Figure 9, a computer as an information processing apparatus 100 of the example embodiment 2 further comprises an analysis result correcting part 150. The analysis result correcting part 150 corrects an analysis result in a manner that the read number of the read sequence determined as the error sequence by the determination part 140 is added to a read number of the a sequence regarded as the true sequence.

Herein, the process by the analysis result correcting part 150 have a common concept with a technology referred to as "deblur" in a field of image processing. That is, in the technology referred to as "deblur", unclear image may be corrected to its original image under a situation where Point spread function is known, which indicates how one point has been spread. Herein, if the "one point" is regarded as the "true sequence", "how one point has been spread" is regarded as the "error sequence", and the "Point spread function" is regarded as the generation probability", the technology referred to as "deblur" may be applied to the process by the analysis result correcting part 150. Herein, with respect to "deblur", see also Tokuhyo No. 2017-531244, and the like.

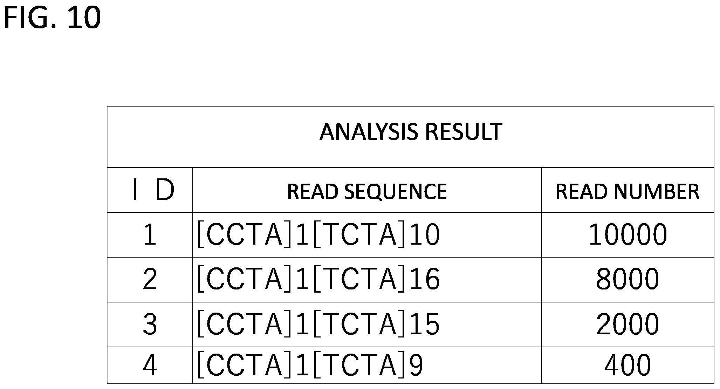

An effect by the information processing apparatus 100 of the example embodiment 2 is conceptually explained while referring to a concrete example. For example, a premise is provided, in which a sample was obtained from one person and an analysis result regarding D1S1656 was obtained as illustrated in Figure 10. Under such premise, according to the information processing apparatus 100 of the example embodiment 1, when the ID: 2 is regarded as the true sequence, the read sequence of ID: 3 is determined as the error sequence. In addition, when ID: 1 is regarded as the true sequence, the read sequence of ID: 4 is determined as the error sequence. That is, the read sequences of IDs: 1, 2 are determined as the true sequences, and the read sequences of IDs: 3, 4 are determined as the error sequences. Herein, the read numbers of ID: 1 and ID: 2 have significant difference. That is, they have imbalanced peak height, thus it is impossible to determine that ID: 1 and ID: 2 are derived from one person.

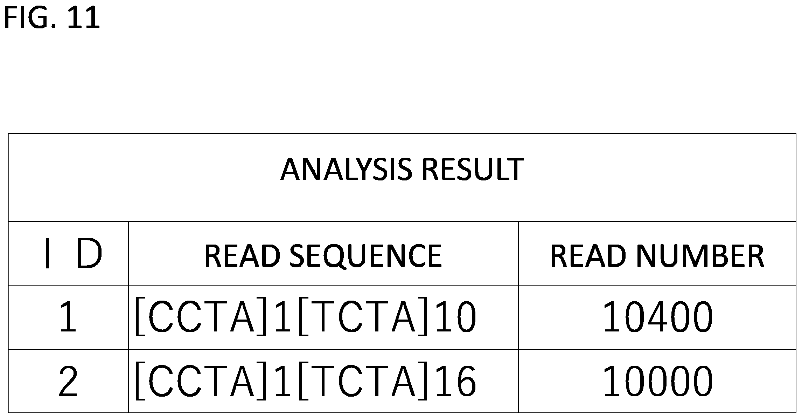

In the information processing apparatus 100 of the example embodiment 2, the analysis result correcting part 150 corrects the analysis result illustrated in Figure 10 to an analysis result illustrated in Figure 11. That is, it is assumed that the error sequence of ID: 3 is the stutter sequence incorrectly amplified upon PCR amplification of the true sequence of ID: 2, thus, under the assumption that all of the true sequence of ID: 2 would have been correctly amplified, the read number of ID: 2 would be 8000 + 2000. In addition, assumedly the error sequence of ID: 4 would be the stutter sequence incorrectly amplified upon PCR amplification of the true sequence of ID: 1, thus under the assumption that all of the true sequence of ID: 1 would have been correctly amplified, the read number of ID: 2 would be 10000+ 400. As described above, the analysis result correcting part 150 corrects the analysis result to indicate the read number of a case where all true sequences are assumed to be correctly amplified.

In the corrected analysis result illustrated in Figure 11, the read numbers of ID: 1 and ID: 2 are balanced. As a result, it may be determined that ID: 1 and ID: 2 are derived from the same person (i.e., a person whose D1S1656 is heterozygote).

As described above, according to the information processing apparatus 100 of the example embodiment 2, the read number of the error sequence incorrectly amplified upon PCR amplification is added to the read number of the true sequence, thus peak height balance is improved. As a result, according to the information processing apparatus 100 of the example embodiment 2, reliability in DNA profiling is improved for a profile regarding a person having heterozygote.

A part or all of the example embodiments are described as the following modes, but not limited thereto.

### (Mode 1)

An information processing apparatus, comprising:
a storage part that stores, for each of isoalleles of a microsatellite which are identified in DNA profiling, a true sequence correctly amplified by PCR, an error sequence incorrectly amplified upon PCR, and a generation probability of the error sequence in association with each other;
an analysis result acquiring part that acquires an analysis result in which read sequences which are read by subjecting a sample to PCR and sequence analysis and read numbers of the read sequences are listed in association with each other;
a prospect part that refers to the storage part while regarding the read sequences as a true sequence for each of the read sequences listed in the analysis result so as to acquire an associated error sequence as a prospected error sequence, and obtains a value as a prospected read number by multiplying the generation probability of the associated error sequence with the read number of each of the read sequences;
a determination part that retrieves a read sequence identical with the prospected error sequence among the read sequences listed in the analysis result, and determines that a retrieved read sequence as an error sequence in a case where the read number of the retrieved read sequence matches with the prospected read number.

### (Mode 2)

The information processing apparatus according to Mode 1, wherein the determination part determines a read sequence which is not determined as the error sequence among the read sequences listed in the analysis result as a true sequence.

### (Mode 3)

The information processing apparatus according to Mode 1 or 2, further comprising an analysis result correcting part that corrects the analysis result in a manner that the read number of the read sequence determined as the error sequence by the determination part is added to the read number of the read sequence regarded as a true sequence.

### (Mode 4)

The information processing apparatus according to any one of Modes 1 to 3, wherein the error sequence is: a stutter sequence in which repeat number is increased or reduced when compared with an original sequence; an indel sequence in which one or more nucleotide base is inserted into/deleted from an original sequence; and/or a nucleotide substitution sequence in which at least one nucleotide base in an original sequence is substituted with another nucleotide base.

### (Mode 5)

An information processing method, including:
an analysis result acquiring step of acquiring an analysis result in which read sequences which are read by subjecting a sample to PCR and sequence analysis and read numbers of the read sequences are listed in association with each other;
a prospect step of referring to a storage part that stores, for each of isoalleles of a microsatellite which are identified in DNA profiling, a true sequence correctly amplified by PCR, an error sequence incorrectly amplified upon PCR, and a generation probability of the error sequence in association with each other, while regarding the read sequences as a true sequence for each of the read sequences listed in the analysis result so as to acquire an associated error sequence as a prospected error sequence, and obtaining a value as a prospected read number by multiplying the generation probability of the associated error sequence with the read number of each of the read sequences;
a determination step of retrieving a read sequence identical with the prospected error sequence among the read sequences listed in the analysis result, and determining that a retrived read sequence as an error sequence in a case where the read number of the retrived read sequence matches with the prospected read number.

### (Mode 6)

An information processing program causing a computer to execute:
an analysis result acquiring process of acquiring an analysis result in which read sequences which are read by subjecting a sample to PCR and
sequence analysis and read numbers of the read sequences are listed in association with each other;
a prospect process of referring to a storage part that stores, for each of isoalleles of a microsatellite which are identified in DNA profiling, a true sequence correctly amplified by PCR, an error sequence incorrectly amplified upon PCR, and a generation probability of the error sequence in association with each other, while regarding the read sequences as a true sequence for each of the read sequences listed in the analysis result so as to acquire an associated error sequence as a prospected error sequence, and obtaining a value as a prospected read number by multiplying the generation probability of the associated error sequence with the read number of each of the read sequences;
a determination process of retrieving a read sequence identical with the prospected error sequence among the read sequences listed in the analysis result, and determining that a retrived read sequence as an error sequence in a case where the read number of the retrived read sequence matches with the prospected read number.

Herein, it is considered that the disclosures of the above Patent Literatures and cited literatures are incorporated herein by reference thereto, and the disclosures may be used as a base or a part of the disclosure as necessary. Variations and adjustments of the example embodiments and examples are possible within the ambit of the entire disclosure (including the claims) of the disclosure and based on the basic technical concept thereof. In addition, various combinations and selections (including non-selection) of various disclosed elements (including each element in each claim, each example embodiment, each drawing, etc.) are possible within the ambit of claims of the disclosure. Namely, the disclosure of course includes various variations and modifications that could be made by those skilled in the art according to the overall disclosure including the claims and the technical concept. Further, each of the disclosed matters of the above cited literatures is regarded as included in the described matters in the application, if required, on the basis of the concept of the disclosure, as a part of the disclosure, also that a part or entire thereof is used in combination with a described matter(s) in the application.

### REFERENCE SIGNS LIST

- 100: information processing apparatus
- 110: storage part
- 120: analysis result acquiring part
- 130: prospect part
- 140: determination part
- 150: analysis result correcting part

## Claims

1. An information processing apparatus, comprising:
a storage part that stores, for each of isoalleles of a microsatellite which are identified in DNA profiling, a true sequence correctly amplified by PCR, an error sequence incorrectly amplified upon PCR, and a generation probability of the error sequence in association with each other;
an analysis result acquiring part that acquires an analysis result in which read sequences which are read by subjecting a sample to PCR and sequence analysis and read numbers of the read sequences are listed in association with each other;
a prospect part that refers to the storage part while regarding the read sequences as a true sequence for each of the read sequences listed in the analysis result so as to acquire an associated error sequence as a prospected error sequence, and obtains a value as a prospected read number by multiplying the generation probability of the associated error sequence with the read number of each of the read sequences;
a determination part that retrieves a read sequence identical with the prospected error sequence among the read sequences listed in the analysis result, and determines that a retrieved read sequence as an error sequence in a case where the read number of the retrieved read sequence matches with the prospected read number.

2. The information processing apparatus according to Claim 1, wherein the determination part determines a read sequence which is not determined as the error sequence among the read sequences listed in the analysis result as a true sequence.

3. The information processing apparatus according to Claim 1 or 2, further comprising an analysis result correcting part that corrects the analysis result in a manner that the read number of the read sequence determined as the error sequence by the determination part is added to the read number of the read sequence regarded as a true sequence.

4. The information processing apparatus according to any one of Claims 1 to 3, wherein the error sequence is: a stutter sequence in which repeat number is increased or reduced when compared with an original sequence; an indel sequence in which one or more nucleotide base is inserted into/deleted from an original sequence; and/or a nucleotide substitution sequence in which at least one nucleotide base in an original sequence is substituted with another nucleotide base.

5. An information processing method, including:
an analysis result acquiring step of acquiring an analysis result in which read sequences which are read by subjecting a sample to PCR and sequence analysis and read numbers of the read sequences are listed in association with each other;
a prospect step of referring to a storage part that stores, for each of isoalleles of a microsatellite which are identified in DNA profiling, a true sequence correctly amplified by PCR, an error sequence incorrectly amplified upon PCR, and a generation probability of the error sequence in association with each other, while regarding the read sequences as a true sequence for each of the read sequences listed in the analysis result so as to acquire an associated error sequence as a prospected error sequence, and obtaining a value as a prospected read number by multiplying the generation probability of the associated error sequence with the read number of each of the read sequences;
a determination step of retrieving a read sequence identical with the prospected error sequence among the read sequences listed in the analysis result, and determining that a retrived read sequence as an error sequence in a case where the read number of the retrived read sequence matches with the prospected read number.

6. An information processing program causing a computer to execute:
an analysis result acquiring process of acquiring an analysis result in which read sequences which are read by subjecting a sample to PCR and sequence analysis and read numbers of the read sequences are listed in association with each other;
a prospect process of referring to a storage part that stores, for each of isoalleles of a microsatellite which are identified in DNA profiling, a true sequence correctly amplified by PCR, an error sequence incorrectly amplified upon PCR, and a generation probability of the error sequence in association with each other, while regarding the read sequences as a true sequence for each of the read sequences listed in the analysis result so as to acquire an associated error sequence as a prospected error sequence, and obtaining a value as a prospected read number by multiplying the generation probability of the associated error sequence with the read number of each of the read sequences;
a determination process of retrieving a read sequence identical with the prospected error sequence among the read sequences listed in the analysis result, and determining that a retrived read sequence as an error sequence in a case where the read number of the retrived read sequence matches with the prospected read number.
